# EUROPÄISCHE PATENTSCHRIFT

(11) **EP 1 321 114 B1**
(45) Veröffentlichungstag und Bekanntmachung des Hinweises auf die Patenterteilung: **07.03.2018**
(21) Anmeldenummer: 02018730.8
(22) Anmeldetag: 21.08.2002
(51) Int. Cl.: A61F 2/40

(54) **Schultergelenkprothese**
Shoulder prothesis
Prothèse de l'épaule

(30) Priorität: 21.11.2001 EP 01811120
(43) Veröffentlichungstag der Anmeldung: 25.06.2003
(73) Patentinhaber: Zimmer GmbH, 8404 Winterthur (CH)
(72) Erfinder: Rauscher, Markus, 6319 Allenwinden (CH); Wendt, Peter, 8542 Wiesendangen (CH)
(74) Vertreter: Manitz Finsterwald Patentanwälte PartmbB

(56) Entgegenhaltungen:
- EP-A- 0 679 375
- EP-A- 0 953 321
- WO-A-97/25943
- US-B1- 6 197 063

## Beschreibung

Die Erfindung handelt von einer Schultergelenkprothese mit zwei aufeinander gleitenden Lagerkörpern, die zum Oberarm mit einem Schaft respektive zum Schulterknochen mit einer Plattform verbindbar sind.

Eine derartige Prothese ist in der Patentanmeldung WO 97/25943 gezeigt. Ein mit einem Schaft im Oberarm verbindbarer Lagerkörper besitzt an seiner Unterseite einen vorstehenden Konus mit Kreisquerschnitt und Indexierbohrungen für einen aus dem Schaft vorstehenden Stift, um in verschiedenen Winkelstellungen den Lagerkörper mit seinem Kreiskonus festzusetzen. Bei dieser Anordnung müssen die Indexierbohrungen und der vorstehende Stift ein Mindestspiel zueinander aufweisen, damit Konus und Gegenfläche mit Sicherheit eine Verbindung bilden. Wegen dieses Spiels werden an der Verbindung auch Torsionskräfte übertragen, die nur durch die Haftreibung übertragen werden können und die zusätzlich zu den axialen Belastungen an den Reibstellen auftreten. Ein derartiger Reibschluss ist mit den zusätzlich auftretenden Schubspannungen durch Torsionsbelastung als Verbindung mehr gefährdet, wobei eine Streuung der übertragbaren Kräfte schon aus dem Schlag, mit welchem ein Press-fit erzeugt werden soll, und aus dem Zustand auf den Konusoberflächen entsteht.

EP 0 953 321 A1 beschreibt eine Schultergelenkprothese mit einem Lagerkörper, der zum Oberarm mit einem Schaft verbindbar ist. Die Verbindung zum Schaft erfolgt durch einen nicht rotationssymmetrischen Körper mit einem selbsthemmenden Sitz, wobei der Umfang des Körpers einen zu einer Längsachse drehfesten und verkeilten Formschluss mit einer Gegenform im Schaft bildet.

Es ist daher Aufgabe der vorliegenden Erfindung eine bessere Verbindung für eine Befestigung am Schaft einer Schultergelenkprothese zu schaffen. Diese Aufgabe wird mit den Kennzeichen des unabhängigen Anspruchs 1 gelöst, indem die Verbindung zum Schaft durch einen nicht rotationssymmetrischen konischen Körper mit einem selbsthemmenden Sitz erfolgt, wobei dessen Umfang einen zu einer Längsachse drehfesten und durch die Konizität verkeilten Formschluss mit einer den Konischen körper (7) zweidimensional umfassenden Gegenform im Schaft bildet, damit die Verbindung lösbar und in gleicher Winkellage wiederholt festsetzbar ist.

Eine solche Anordnung hat den Vorteil, dass bezüglich der Längsachse der Verbindung in beiden Drehrichtungen bereits Anteile von Normalkräften durch die Verspannung im Ruhezustand vorhanden sind, die bei einer Torsionsbelastung entsprechend dem Abstand ihrer Wirkungslinie zur Drehachse entgegen wirken können. Ein weiterer Vorteil besteht darin, dass durch die Abweichung vom Kreisquerschnitt der Konusverbindung bewusst Auflagepunkte gewählt werden können, die an der äußeren konischen Hülse keine nur von Ringspannung und Elastizität des Materials abhängige Aufweitung erfahren, sondern eine zusätzliche Biegebelastung an der Hülse erzeugen, was einer weicheren Federwirkung der Hülse entspricht und damit einen größeren Aufschiebeweg und mehr Sicherheit für eine erreichte Haltekraft ergibt. Weitere vorteilhafte Fortbildungen der Erfindung ergeben sich durch die abhängigen Ansprüche 2 bis 13. So ist es vorteilhaft, bei einem elliptischen Umfang des konischen Körpers die angestrebten Berührungspunkte so zu wählen, dass sie in ihrem radialen Abstand zur Längsachse zwischen dem Betrag der großen und kleinen Halbachse der Ellipse, aber näher bei dem der großen Halbachse liegen. Ähnlich ist es bei einem Dreibogengleichdick, bei dem die Berührungspunkte mit ihrem radialen Abstand zur Längsachse zwischen dem Betrag des kleinsten und größten Bogenabstandes, aber näher bei dem des größten Bogenabstandes liegen sollten. Gut sind grundsätzlich Berührungspunkte, in denen die Normalkraft mit ihrer Wirkungslinie einen großen senkrechten Abstand zur Längsachse aufweist.

Die Stärke der konischen Verbindung, die wiederholt lösbar ist, gestattet es auch in Abwandlung zum natürlichen Schultergelenk an einem bereits implantierten Schaft einen konischen Körper mit einer Kugelschale anzubringen, welche auf einem an der Plattform angebrachten Kugelkopf schwenkbar gelagert ist. Diese Umkehr des Gelenks, die weniger Gleiten und hauptsächlich ein Schwenken um den Kugelmittelpunkt zulässt, ist notwendig, wenn die Bänder stark geschädigt sind. Die Stärke der konischen Verbindung gestattet es, auch komplexere Anwendungen, wie sie in der Patentanmeldung FR 2727857 gezeigt sind, einzubeziehen. In dieser Publikation sind Ausführungsbeispiele mit einem Schaft gezeigt, der als längsgeschlitzte Kugel mit konischer Bohrung endet, um diese über einen konischen Druckkörper in einer kugelförmigen Ausnehmung des Lagerkopfes festzusetzen. Dadurch, dass diese Kugel an den konischen Körper der vorliegenden Erfindung angeformt ist und dieser auch eine Verstellschraube und den konischen Druckkörper aufnimmt, kann zunächst der Schaft implantiert werden, dann die beste Winkellage des Lagerkopfes mit einer als konischer Körper aufgesteckten Manipulierprothese festgelegt werden und unabhängig vom bereits implantierten Schaft die gleiche Winkellage an einem gleich großen Lagerkopf eingestellt werden. Eine solche Winkellage kann zusätzlich durch einen vom konischen Körper vorstehenden Stift mit einer dazu passenden Bohrung in der kugelförmigen Aufnahme des Lagerkopfes gesichert werden.

Eine weitere Möglichkeit für das Festsetzen des Kugelgelenks im Lagerkörper besteht in einem entgegengesetzt geschlitzten Kugelkörper mit konischer Bohrung, der auf einen zusätzlichen am konischen Körper angeformten Kreiskonus aufsteckbar ist und der durch einen Schlag auf den aufgesetzten Lagerkörper in diesem und selbsthemmend am konischen Körper festsetzbar ist.

Eine weitere Verbesserung der konischen Verbindung zwischen konischem Körper und der Gegenform im Schaft wird erreicht, wenn die Berührungspunkte in zwei zur Längsachse quer stehenden Ebenen verteilt sind und die beiden Ebenen einen vorgegebenen Mindestabstand zueinander aufweisen. Dies wird beispielsweise durch eine Unterbrechung der Eingriffsfläche im mittleren Bereich des konischen Körpers erreicht.

Im folgenden wird die Erfindung anhand von Ausführungsbeispielen beschrieben. Es zeigen:
- Fig. 1:: schematisch einen im Oberarmknochen implantierten Schaft;
- Fig. 2:: schematisch ein künstliches Schultergelenk mit einem Lagerkopf, der einen zum Schaft von Fig. 1 passenden konischen Körper aufweist;
- Fig. 3:: schematisch einen Querschnitt durch einen konischen Körper gemäß Fig. 2 mit einem elliptisch verlaufenden Umfang;
- Fig. 4:: schematisch einen Querschnitt durch einen konischen Körper gemäß Fig. 2, bei dem der Umfang einem Dreibogengleichdick entspricht;
- Fig. 5:: schematisch im Schnitt einen Lagerkörper, der durch einen Schlag über ein festsetzbares Kugelgelenk mit einem konischen Körper gemäß Fig. 2 verbinden lässt;
- Fig. 6:: schematisch eine Ansicht auf einen Kugelkörper und den konischen Körper von Fig. 5;
- Fig. 7:: schematisch im Schnitt einen Lagerkörper, bei dem ein Kugelgelenk zwischen Lagerkörper und einem konischen Körper mit einer in der Achsrichtung des konischen Körpers verlaufenden Schraube festsetzbar ist;
- Fig. 8:: schematisch einen in einem Oberarmknochen implantierten Schaft mit einer Gegenform für einen konischen Körper;
- Fig. 9:: schematisch einen zum Schaft von Fig. 8 passenden konischen Körper, der mit einer Kugelschale ein künstliches Schultergelenk zu einem Kugelkopf bildet, der über eine Plattform am Schulterknochen befestigt ist;
- Fig. 10:: schematisch einen Ausschnitt von Fig. 7 mit einem Druckkörper, der einen stiftförmigen Fortsatz für eine zusätzliche Sicherung im Lagerkörper aufweist;
- Fig. 11:: schematisch einen Probierlagerkopf für eine Anordnung gemäss Fig. 7;
- Fig. 12:: schematisch einen Schnitt gemäß Fig. 3, an dem eine drehfeste Verkeilung zwischen konischem Körper und Gegenform dargestellt ist;
- Fig. 13:: schematisch einen Schnitt durch einen konischen Körper mit einem ursprünglichen Rechteckquerschnitt, an dem nachträglich konische Teilflächen, welche im Schnitt elliptisch und passend zu einer Gegenform gemäß Fig. 12 hergestellt wurden; und
- Fig. 14:: schematisch einen konischen Körper gemäß Fig. 6, bei dem im mittleren Bereich der konischen Fläche eine Unterbrechung eingearbeitet ist.

In den nachfolgenden Ausführungsbeispielen sind gleiche Hinweiszeichen für gleiche Funktionen verwendet.

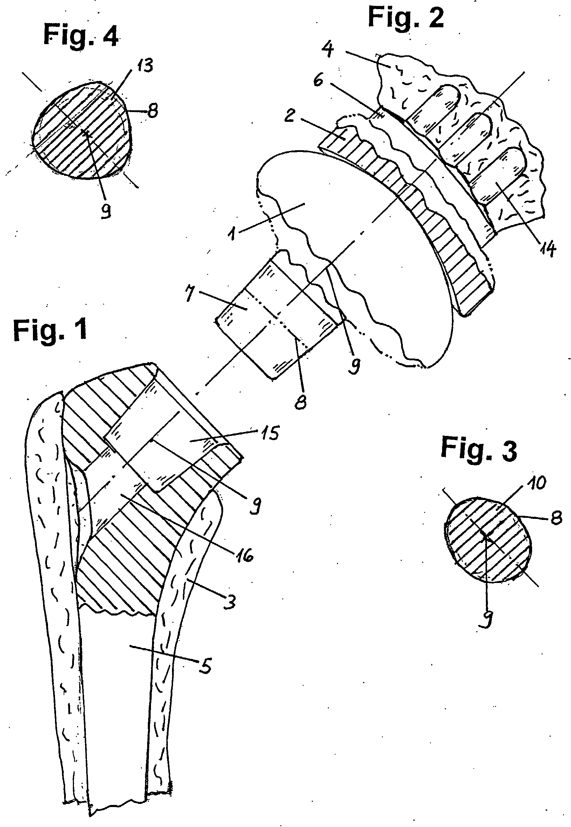

Ein erstes Ausführungsbeispiel ist in den Fig. 1 und 2 gezeigt. Ein Schaft 5 ist in einem Oberarmknochen 3 implantiert, wobei der Schaft 5 direkt in einem vorbereiteten Knochenbett verankert ist. Der Schaft kann aber ebensogut ein mit Knochenzement im Oberarmknochen verankerter Schaft sein. In Richtung einer Längsachse 9 für das eigentliche Schultergelenk ist eine Bohrung 16 angebracht, die in einer Gegenform 15 für einen konischen Körper 7 endet. Das eigentliche Gelenk wird durch einen mit dem konischen Körper 7 starr verbundenen Lagerkopf 1 und eine Lagerschale 2 gebildet, die ihrerseits mit einer im Schulterknochen 4 verankerten Plattform 6 starr verbunden ist. Zur Verankerung der Plattform 6 sind parallel zueinander Zapfen 14 an der Plattform 6 angebracht, die beispielsweise mit Knochenzement oder durch Presssitz in vorbereiteten Bohrungen des Schulterknochens 4 verankert sind.

Der konische Körper 7, und entsprechend die Gegenform 15, besitzt einen Querschnitt 10 mit Umfang 8, der gemäß Fig. 3 elliptisch ausgebildet ist. In Fig. 4 ist ein aus einem gleichseitigen Dreieck abgeleiteter Querschnitt gezeigt, der einem Dreibogengleichdick 13 entspricht, einer Form, die im Maschinenbau als Wellenverbindung verwendet wird.

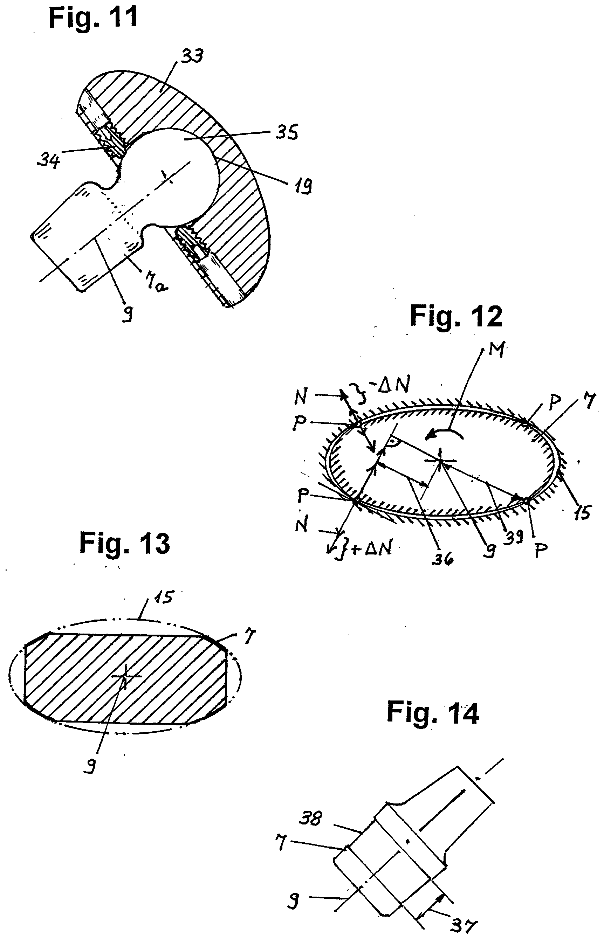

In Fig. 12 sind die angestrebten Verhältnisse bei einem annähernd elliptischen Querschnitt gezeigt. Es sind durch geringfügige Formabweichungen zwischen konischem Körper 7 und der Gegenform 15 vier Kontaktpunkte P vorgesehen, die sich bei intensiver Pressung zu Kontaktflächen erweitern. Ein radialer Abstand 39 eines Kontaktpunktes P ist so gewählt, dass eine Normalkraft N in einem Kontaktpunkt P mit ihrer Wirkungslinie in einem relativ großen Abstand 36 an der Längsachse 9 vorbeiführt, um Anteile von einem Drehmoment M als Veränderungen von Normalkräften zu übertragen. So wird ein zusätzlich am konischen Körper 7 angreifendes Drehmoment M durch eine Abnahme der Vorspannung resp. durch eine Erhöhung der Vorspannung N um einen Bruchteil ΔN kompensiert. Der vorgespannte lokale Formschluss ist also entscheidend. In Fig. 13 ist die gleiche Situation extremer dargestellt. Der konische Körper 7 ist nur noch im Bereich der zu Kontaktflächen erweiterten Kontaktpunkte mit der elliptischen Grundform 15 in Berührung. Die restlichen Flächen sind zurückgesetzt.

Eine weitere Möglichkeit für die Abänderung des konischen Körpers 7 ist in Fig. 14 dargestellt. Um möglichst große Biegemomente in der Längsachse 9 übertragen zu können, findet die Verspannung in zwei Querschnitten statt, die um einen Mindestabstand 37 auseinanderliegen. Das heißt, der Konus hat im mittleren Bereich eine Unterbrechung 38 von diesem Mindestabstand 37.

Bei einem Dreibogengleichdick, wie es in Fig. 4 gezeigt ist, sind im Rahmen der Fertigungstoleranzen ebenfalls mehr als drei Kontaktpunkte angestrebt, wobei einige Kontaktpunkte mit den Wirkungslinien ihrer Normalkräfte ebenfalls in einem Abstand zur Längsachse 9 liegen sollten.

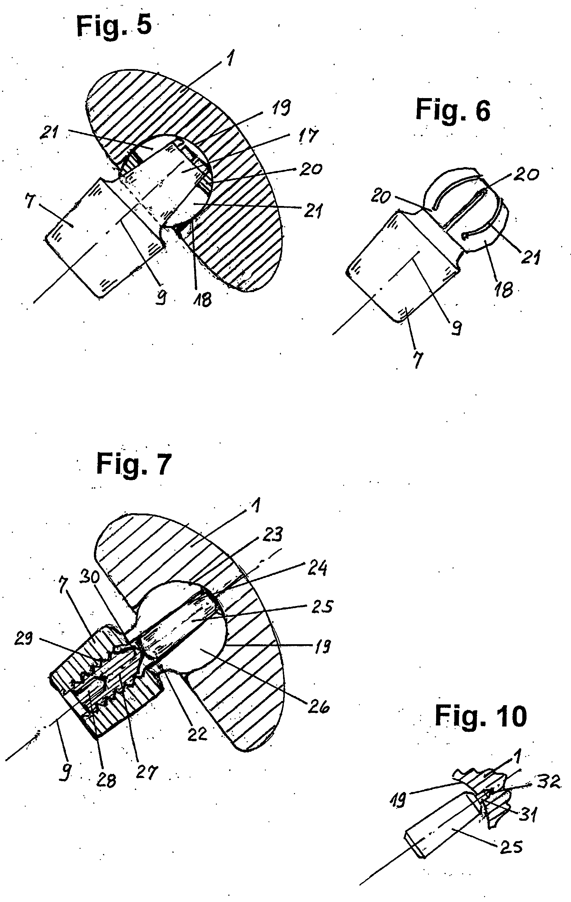

Im Beispiel der Fig. 5 und 6 ist die starre Verbindung zwischen konischem Körper 7 und Lagerkopf 1 durch ein festsetzbares Kugelgelenk realisiert. Der Lagerkopf besitzt eine kugelförmige Ausnehmung 19, die im Längsschnitt einen Winkel von mehr als 180° einschließt. In diese Ausnehmung 19 ist ein komprimierbarer Kugelkörper 18 einsetzbar. Dieser ist komprimierbar, weil er eine durchgehende Innenbohrung und wechselseitig angebrachte Schlitze 21 aufweist, die an den Stirnseiten schmale Brücken 20 stehen lassen. Da die Innenbohrung als Kreiskonus ausgeführt ist, lässt sich der Kugelkörper 18 durch einen passenden Kreiskonus 17, der am konischen Körper 7 angeformt ist, aufspreizen. Dieser Kreiskonus 17 ist mit seinem Konuswinkel selbsthemmend gewählt, was zur Folge hat, dass bei einem Schlag auf den Lagerkopf 1 oder auf den konischen Körper 7 in Richtung der Längsachse 9 der Lagerkopf 1 gegenüber dem Kugelkörper 18 und der Kugelkörper 18 gegenüber dem konischen Körper 7 festgesetzt wird.

Ein weiteres Beispiel für ein festsetzbares Kugelgelenk zwischen Lagerkopf 1 und konischem Körper 7 ist in Fig. 7 gezeigt. An den konischen Körper 7 ist ein Hals 22 und eine Kugel 23 angeformt. Die Kugel 23 besitzt eine konische Bohrung 24 und Längsschlitze, die vom Scheitel bis in den Hals 22 gehen, sodass der Hals 22 in viele Biegeelemente aufgeteilt ist. Solange die konische Bohrung 24 leer ist, lässt sich die Kugel 23 zusammenpressen und in die kugelförmige Ausnehmung 19 des Lagerkopfes 1 einfahren. Erst wenn durch eine Gewindebohrung 29 im konischen Körper 7 ein konischer Druckkörper 25 eingeschoben und durch eine Schraube 27 vorwärts getrieben wird, können die durch Längsschlitze 26 getrennten Lamellen der Kugel 23 auseinandergespreizt werden, wobei die Halspartie eine Art Biegefeder und Fliessgelenk bildet. Statt der Schraube 27 kann auch ein Stössel eingeschoben werden, um mit einem Schlag auf den Stössel den konischen Druckkörper 25 festzusetzen. Die Schraube 27 ist als Madenschraube mit einem Innensechskant 28 versehen und treibt den konischen Druckkörper 25 mit einer Nase 30 vorwärts. Da wegen der kurzen Abmessungen im Halsbereich auch plastische Verformungen eintreten können, ist nur ein einmaliger Setzvorgang vorgesehen. Zu diesem Zweck werden gemäß Fig. 11 ein konischer Körper 7a und ein Probierlagerkopf 33, die äußerlich baugleich sind, im Schaft 5 (Fig. 1 und 2) eingesetzt, um eine optimale Stellung für den Probierlagerkopf 33 zu finden. Der konische Körper 7a besitzt einen starren Kugelkopf 35, an dem Madenschrauben 34 angreifen, um den Probierlagerkopf 33 in seiner optimalen Stellung zu fixieren. Anschließend werden der konische Körper 7a und der Probierlagerkopf 33 vom Schaft 5 gelöst, um außerhalb des Operationsfeldes die Lage vom Probierlagerkopf 33 zum konischen Körper 7a auf den endgültigen konischen Körper 7 und seinen Lagerkopf 1 (Fig. 7) zu kopieren. Wie so ein Kopiervorgang zur Erreichung einer gleichen relativen Lage aussehen kann, ist in der Patentanmeldung EP-A-0 931 522 gezeigt, wobei jedoch nur der vom Schaft lösbare konische Körper 7, 7a in der gleichen Vorrichtung eingespannt werden muss. Gemäß Fig. 10 kann in der optimalen Stellung eine Aussparung 32 in der kugelförmigen Ausnehmung 19 des Lagerkopfes angebracht sein, in welche ein stiftförmiger Fortsatz 31 des konischen Druckkörpers 25 als zusätzliche Drehsicherung hineinragt.

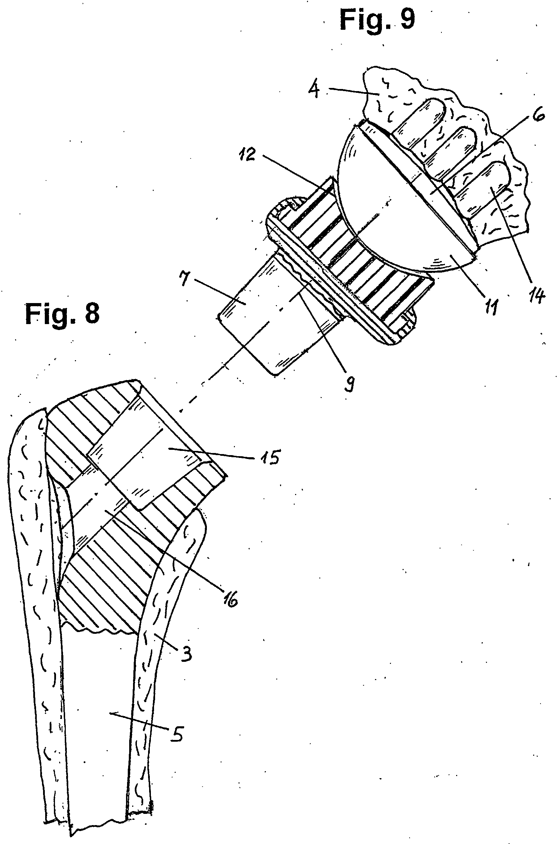

Im Beispiel von Fig. 8 und 9 sind die Funktionen von Kugel und Lagerschale vertauscht, um den Oberarm um einen Drehpunkt drehen zu lassen. Der im Oberarmknochen 3 implantierte Schaft 5 ist wiederum mit einer Bohrung 16 und mit einer Gegenform 15 für einen konischen Körper 7 versehen. Der konische Körper 7 ist jedoch verbreitert zu einer Aufnahme für eine Kugelschale 12, die ihrerseits einen Kugelkopf 11 teilweise umschließt. Der Kugelkopf 11 ist durch eine Schnappverbindung oder Schraubverbindung (beide nicht gezeigt) auf einer Plattform 6 befestigt, die über Zapfen 14 im Schulterknochen 4 verankert ist. Die Verankerung der Plattform 6 kann ebenso über Knochenschrauben und vorstehende Rippen im Schulterknochen erfolgen.

In Fig. 14 ist ein konischer Körper 7 gezeigt, bei dem der tragende konische Teil eine Unterbrechung 38 aufweist. Auf diese Weise entstehen zwei konische Bereiche für die Verklemmung zu einer Gegenform 15 (nicht gezeigt), wobei diese Bereiche um einen Mindestabstand 37 auseinanderliegen, um Biegemomente in der Längsachse 9 übertragen zu können.

Festigkeitsberechnungen und praktische Versuche haben gezeigt, dass bei einer Anordnung mit einem elliptischen Querschnitt des konischen Körpers 7 und seiner Gegenform 15 eine optimale Ausnutzung des Materials dann stattfindet, wenn die Ellipse in ihrer Ebene so ausgerichtet ist, dass ihre große Achse in einer Projektion gegen lateral als Senkrechte erscheint. Eine derartige Anordnung erlaubt es, bei einer von anterior nach posterior begrenzten Breite vom Schaft 5 ein Maximum an Festigkeit für die konische Klemmverbindung zwischen konischem Körper 7 und seiner Gegenform 15 zu erreichen. Dies gilt für Anordnungen mit vollem konischen Körper 7 gemäß Figuren 2, 5 und 9 wie auch für einen konischen Körper 7 mit Bohrung 29 gemäß Figur 7, solange der Schaft quer zu der Längsachse 9 eine geringere Dicke von posterior nach anterior als in anderen Richtungen aufweist.

## Patentansprüche

1. Schultergelenkprothese mit zwei aufeinander gleitenden Lagerkörpern (1, 2; 11, 12) die zum Oberarm (3) mit einem Schaft (5) respektive zum Schulterknochen (4) mit einer Plattform (6) verbindbar sind, **dadurch gekennzeichnet, dass** die Verbindung zum Schaft (5) durch einen nicht rotationssymmetrischen, konischen Körper (7) mit einem selbsthemmenden Sitz erfolgt, wobei dessen Umfang (8) einen zu einer Längsachse (9) drehfesten und durch die Konizität verkeilten Formschluss mit einer den Konischen Körper (7) zweidimensional umfassenden Gegenform (15) im Schaft (5) bildet, damit die Verbindung lösbar und in gleicher Winkellage wiederholt festsetzbar ist.

2. Schultergelenkprothese nach Anspruch 1, **dadurch gekennzeichnet, dass** der Umfang (8) des konischen Körpers (7) und der Gegenform (15) elliptisch (10) ausgebildet ist.

3. Schultergelenkprothese nach Anspruch 1, **dadurch gekennzeichnet, dass** der Umfang (8) des konischen Körpers (7) und der Gegenform (15) als Dreibogengleichdick (13) ausgeführt ist.

4. Schultergelenkprothese nach einem der Ansprüche 1 bis 3, **dadurch gekennzeichnet, dass** an der Plattform (6) eine Lagerschale (2) und am Schaft (5) ein Lagerkopf (1) befestigt ist.

5. Schultergelenkprothese nach Anspruch 4, **dadurch gekennzeichnet, dass** der Lagerkopf (1) über ein festsetzbares Kugelgelenk mit dem konischen Körper (7) verbunden ist.

6. Schultergelenkprothese nach Anspruch 5, **dadurch gekennzeichnet, dass** an den konischen Körper (7) eine längsgeschlitzte (26) Kugel (23) mit einer konischen Bohrung (24) angeformt ist und dass der konische Körper (7) eine daran anschließende Gewindebohrung (29) aufweist, um mit einer Schraube (27) und mit einem konischen Druckkörper (25) die Kugel (23) auseinander zu spreizen und in einer kugelförmigen Ausnehmung (19) des Lagerkopfes (1) festzusetzen.

7. Schultergelenkprothese nach Anspruch 6, **dadurch gekennzeichnet, dass** der konische Druckkörper (25) in seiner Endstellung mit einem stiftförmigen Fortsatz (31) als zusätzliche Drehsicherung in eine Aussparung (32) des Lagerkopfes (1) eingreift.

8. Schultergelenkprothese nach Anspruch 5, **dadurch gekennzeichnet, dass** der konische Körper (7) einen zusätzlichen Kreiskonus (17) aufweist, auf den ein aufspreizbarer Kugelkörper (18) selbsthemmend aufsteckbar ist, welcher in einer kugelförmigen Ausnehmung (19) des Lagerkopfes (1) durch Einschlagen vom Kreiskonus (17) verspannbar ist.

9. Schultergelenkprothese nach Anspruch 4, **dadurch gekennzeichnet, dass** die Plattform (6) mit verschieden großen Lagerschalen (2) bestückbar ist.

10. Schultergelenkprothese nach einem der Ansprüche 1 bis 3, **dadurch gekennzeichnet, dass** an der Plattform (6) ein Kugelkopf (11) und am Schaft (5) eine Kugelschale (12) befestigt ist.

11. Schultergelenkprothese nach einem der Ansprüche 1 bis 10, **dadurch gekennzeichnet, dass** am konischen Körper (7) in Längsrichtung gesehen im mittleren Bereich die Kontur etwas zurückgenommen ist, um einen dieser Rücknahme entsprechenden Mindestabstand der Querschnitte zu erreichen, in denen eine Verspannung der konischen Flächen stattfindet.

12. Schultergelenkprothese nach einem der Ansprüche 2 oder 4 bis 11, **dadurch gekennzeichnet, dass** der elliptische Querschnitt des konischen Körpers (7) und seiner Gegenform (15) so in seiner Ebene ausgerichtet ist, dass die große Achse der Ellipse in einer Projektion gegen lateral als Senkrechte erscheint.

13. Schultergelenkprothese nach einem der Ansprüche 1 bis 12, **dadurch gekennzeichnet, dass** die zweidimensionale Umfassung darin besteht, dass zwischen dem konischen Körper (7) und der Gegenform (15) wenigstens drei Kontaktpunkte (P) vorgesehen sind, wobei die Wirkungslinien der Normalkräfte einiger Kontaktpunkte (P) in einem Abstand an der Längsachse (9) vorbeilaufen und einander überschneiden.

## Claims

1. A shoulder joint prosthesis comprising two bearing bodies (1, 2; 11, 12) which slide on one another and which are connectable to the upper arm (3) by a shaft (5) or to the shoulder bone (4) by a platform (6) respectively, **characterized in that** the connection to the shaft (5) takes place by a non-rotationally symmetrical conical body (7) with a self-locking seat, with the periphery (8) of said non-rotationally symmetrical conical body forming a shape match with a counter shape (15) in the shaft (5), which counter shape encompasses the conical body (7) in a two-dimensional manner, said shape match being rotationally fixed with respect to a longitudinal axis (9) and being wedged by the conicity, so that the connection is releasable and can repeatedly be fixed in the same angular position.

2. A shoulder joint prosthesis in accordance with claim 1, **characterized in that** the peripheries (8) of the conical body (7) and of the counter shape (15) are elliptical (10).

3. A shoulder joint prosthesis in accordance with claim 1, **characterized in that** the peripheries (8) of the conical body (7) and of the counter shape (15) are designed as bodies (13) having an outline formed by three curves and of constant diameter.

4. A shoulder joint prosthesis in accordance with any one of the claims 1 to 3, **characterized in that** a bearing shell (2) is fastened to the platform (6) and a bearing head (1) is fastened to the shaft (5).

5. A shoulder joint prosthesis in accordance with claim 4, **characterized in that** the bearing head (1) is connected to the conical body (7) via a ball joint which can be fixed in place.

6. A shoulder joint prosthesis in accordance with claim 5, **characterized in that** a ball (23) slit longitudinally (26) and having a conical bore (24) is molded at the conical body (7); and **in that** the conical body (7) has an adjoining threaded bore (29) in order to spread the ball (23) apart using a screw (27) and using a conical pressure body (25) and to fix it in a spherical recess (19) of the bearing head (1).

7. A shoulder joint prosthesis in accordance with claim 6, **characterized in that** in its end position a pin-shaped prolongation (31) of the conical pressure body (25) engages into a cut-out (32) of the bearing head (1) as an additional security against rotation.

8. A shoulder joint prosthesis in accordance with claim 5, **characterized in that** the conical body (7) has an additional circular cone (17) onto which a ball body (18) can be placed in a self-locking manner, said ball body being able to be spread apart and being able to be clamped in a spherical recess (19) of the bearing head (1) by hammering in the circular cone (17).

9. A shoulder joint prosthesis in accordance with claim 4, **characterized in that** the platform (6) can be fitted with bearing shells (2) of different sizes.

10. A shoulder joint prosthesis in accordance with any one of the claims 1 to 3, **characterized in that** a ball head (11) is fastened to the platform (6) and a ball socket (12) is fastened to the shaft (5).

11. A shoulder joint prosthesis in accordance with any one of the claims 1 to 10, **characterized in that** the contour is somewhat reduced in the middle region at the conical body (7) viewed in the longitudinal direction in order to achieve a minimum spacing of the cross-sections which corresponds to this reduction, with a tensioning of the conical surfaces taking place in said cross-sections.

12. A shoulder joint prosthesis in accordance with any one of the claims 2 or 4 to 11, **characterized in that** the elliptical cross-section of the conical body (7) and of its counter shape (15) is oriented in its plane such that the major axis of the ellipse appears as a perpendicular in a projection toward lateral.

13. A shoulder joint prosthesis in accordance with any one of the claims 1 to 12, **characterized in that** the two-dimensional encompassing comprises at least three contact points (P) being provided between the conical body (7) and the counter shape (15), with the lines of action of the normal forces of some contact points (P) running past the longitudinal axis (9) at a spacing and overlapping one another.

## Revendications

1. Prothèse de l'articulation de l'épaule comprenant deux corps formant palier (1, 2 ; 11, 12) en coulissement l'un contre l'autre, qui sont susceptibles d'être reliés à l'humérus (3) avec une emboîture (5) et respectivement à l'os de l'épaule (4) avec une plate-forme (6), **caractérisée en ce que** la liaison à l'emboîture (5) a lieu au moyen d'un corps conique (7) qui n'est pas à symétrie de révolution, par un engagement en autoblocage, la périphérie de celui-ci (8) formant un blocage à coopération de formes solidaire en rotation par rapport à un axe longitudinal (9) et bloqué grâce à la conicité, avec une forme antagoniste (15), en enserrant le corps conique (7) de façon bidimensionnelle, dans l'emboîture (5), afin que la liaison soit détachable et immobilisable de façon répétée dans la même position angulaire.

2. Prothèse de l'articulation de l'épaule selon la revendication 1, **caractérisée en ce que** la périphérie (8) du corps conique (7) et de la forme antagoniste (15) est réalisée sous forme elliptique (10).

3. Prothèse de l'articulation de l'épaule selon la revendication 1, **caractérisée en ce que** la périphérie (8) du corps conique (7) et de la forme antagoniste (15) est réalisée sous la forme d'une triple courbe de largeur constante (13).

4. Prothèse de l'articulation de l'épaule selon l'une des revendications 1 à 3, **caractérisée en ce qu'**une coque formant palier (2) est fixée sur la plate-forme (6), et **en ce qu'**une tête formant palier (1) est fixée sur l'emboîture (5).

5. Prothèse de l'articulation de l'épaule selon la revendication 4, **caractérisée en ce que** la tête formant palier (1) est reliée au corps conique (7) via une articulation à rotule susceptible d'être immobilisée.

6. Prothèse de l'articulation de l'épaule selon la revendication 5, **caractérisée en ce qu'**une bille (23) fendu en longueur (26) et présentant un perçage conique (24) est conformée sur le corps conique (7), et **en ce que** le corps conique (7) comporte un perçage taraudé (29) qui s'y raccorde, afin de provoquer un écartement de la bille au moyen d'une vis (27) et d'un corps de pressage conique (25) et d'immobiliser la bille dans un évidement (19) de forme sphérique de la tête formant palier (1).

7. Prothèse de l'articulation de l'épaule selon la revendication 6, **caractérisée en ce que**, dans sa position finale, le corps de pressage conique (25) s'engage avec un prolongement en forme de tige (31) à titre de blocage additionnel antirotation dans une échancrure (32) de la tête formant palier (1).

8. Prothèse de l'articulation de l'épaule selon la revendication 5, **caractérisée en ce que** le corps conique (7) comporte un cône circulaire additionnel (17) sur lequel peut être emboîté un corps sphérique (18) susceptible d'être écarté, avec auto coincement, ledit corps sphérique étant susceptible d'être calé dans un évidement de forme sphérique (19) de la tête formant palier (1) en frappant sur le cône circulaire (17).

9. Prothèse de l'articulation de l'épaule selon la revendication 4, **caractérisée en ce que** la plate-forme (6) peut être équipée de coques formant palier (2) de tailles différentes.

10. Prothèse de l'articulation de l'épaule selon l'une des revendications 1 à 3, **caractérisée en ce qu'**une tête sphérique (11) est fixée sur la plate-forme (6) et une coque sphérique est fixée sur l'emboîture (5).

11. Prothèse de l'articulation de l'épaule selon l'une des revendications 1 à 10, **caractérisée en ce que**, dans la région médiane vue en direction longitudinale, le contour du corps conique (7) est légèrement en retrait, afin d'atteindre que les coupes transversales présentent une distance minimum correspondant à ce retrait, dans laquelle se produit une précontrainte des surfaces coniques.

12. Prothèse de l'articulation de l'épaule selon l'une des revendications 2 où 4 à 11, **caractérisée en ce que** la section elliptique du corps conique (7) et sa forme antagoniste (15) est orientée dans son plan de telle façon que le grand axe de l'ellipse apparaît comme vertical dans une projection en sens latéral.

13. Prothèse de l'articulation de l'épaule selon l'une des revendications 1 à 12, **caractérisée en ce que** l'enserrement bidimensionnel résulte du fait qu'il existe au moins trois points de contact (P) entre le corps conique (7) et la forme antagoniste (15), et les lignes d'action des forces normales en quelques points de contact (P) passent à distance devant l'axe longitudinal (9) et se recoupent mutuellement.
